(19) Europäisches Patentamt
European Patent Office
Office européen des brevets

(11) Publication number: 0 274 213 B1

(12) # EUROPEAN PATENT SPECIFICATION

(45) Date of publication of patent specification: **13.05.92** (51) Int. Cl.5: **C07C 247/04**

(21) Application number: **87310476.4**

(22) Date of filing: **27.11.87**

(54) **Process for the preparation of alkyl azide-substituted, hydroxy-terminated polyethers.**

(30) Priority: **01.12.86 CA 524263**

(43) Date of publication of application:
**13.07.88 Bulletin 88/28**

(45) Publication of the grant of the patent:
**13.05.92 Bulletin 92/20**

(84) Designated Contracting States:
**DE FR GB SE**

(56) References cited:
**DE-A- 2 945 383**
**US-A- 4 268 450**
**US-A- 4 486 351**

**Journal of the American Chemical Society,Vol 76,(1954),pages 1231-1235**

(73) Proprietor: **HER MAJESTY IN RIGHT OF CANADA AS REPRESENTED BY THE MINISTER OF NATIONAL DEFENCE**

**Ottawa, Ontario K1A OK2(CA)**

(72) Inventor: **Ahad, Elie**
**3140 Champagne Ste-Foy**
**Ouebec G1W 2Y6(CA)**

(74) Representative: **Lambert, Hugh Richmond**
**D. YOUNG & CO. 10 Staple Inn**
**London, WC1V 7RD(GB)**

EP 0 274 213 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to an improved process for the synthesis of hydroxy-terminated aliphatic polyethers having alkyl azide substituents.

Hydroxy-terminated aliphatic polyethers having alkyl azide substituents are useful as energetic binders and plasticizers in solid propellants and composite explosives. One such polyether is glycidyl azide polymer (GAP). This polymeric azide is used as an energetic binder (at MW 2000 to 6000) and as a plasticizer (at MW of about 500) in composite explosives and solid rocket propellant systems to impart additional energy to the formulations, increase the performance and enhance the stability and the mechanical properties of the system.

A process for the preparation of hydoxy-terminated aliphatic poly-ethers having alkyl azide substituents, e.g. GAP, is described in United States Patent 4,268,450 of 19th May 1981, in the name of M.B. Frankel et al. According to the Frankel at al process, in a first reaction step, the starting material, epichlorohydrin (ECH) is polymerized to polyepichlorohydrin (PECH) using a catalyst, boron trifluoride ($BF_3$) in the presence of a dichloro compound such as carbon dichloride. In a second step, PECH is azidized using a molar excess of the order of 2:1 sodium azide ($NaN_3$) in the presence of a polar organic solvent dimethyl formamide (DMF) for three days at 100 ° C. It is emphasized that a molar excess, of about 2:1, of sodium aside is typically employed. Since sodium azide is of a poisonous nature, the use of large amounts is a distinct advantage. A final purification step using methylene chloride and drying over magnesium sulfate is also described. This multi step process is costly and takes from four to seven days to complete.

In the German counterpart to US Patent 4,268,450, namely DE-A-29 45 383, it is apparently suggested that the two stage reaction can be carried out in the reverse order, i.e. by the initial step of reacting the epichlorohydrin reactant with sodium azide to produce an intermediate of the formula

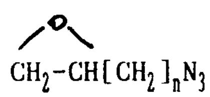

followed by the polymerisation of that intermediate in the presence of a polymerisation catalyst, for example, boron trifluoride. No example is given, and it is to be doubted whether that alternative process is operable in that it is known that, in the reaction of epichlorohydrin and sodium azide, the azide attacks and opens the epoxide ring with great ease to yield a diazido-alcohol as the major product along with unidentified higher and lower boiling components, the alleged epoxidised azide intermediate

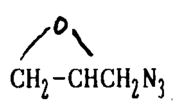

not being identified (Van der Werf et al. J.A.C.S. 76, 1231, 1954).

Be that as it may, the Frankel process as described in US Patent 4,268,450 and DE-A-29 45 383 is essentially a two step process, if only for the reason that two of the materials used are essentially incompatible one with the other and have to be kept apart. These are the boron trifluoride used as the polymerisation catalyst in the first step of the reaction, and the azide reactant used in the second step. The two are totally incompatible since in the presence of humidity $BF_3$ will react with $NaN_3$ to produce hydrazoic acid which is a highly explosive and dangerous chemical. For that reason alone, the Frankel process is the very antithesis of a one step process.

By contrast, the present invention provides an essentially single step process for the production of low molecular weight alkyl azide-substituted hydroxy-terminated aliphatic polyethers, it being understood that, in this context, a single step process refers to a reaction in which the two reactions are carried out concurrently rather than consecutively, notwithstanding that, for control of the exotherm which results when bringing together the epichlorohydrin and aside reactants, the process of the present invention is carried out in two distinct stages, viz: the initial bringing together of the reactants and secondly the subsequent heating of the reaction mixture to complete the reaction - or rather the two concurrent reactions.

In more detail the present invention resides in a process for the preparation of low molecular weight alkyl azide-substituted hydroxy-terminated aliphatic polyethers, i.e. of from 5 to 10 ether units, which comprises

(i) adding sodium, potassium or lithium azide slowly to a reaction mixture containing an alpha-epichlorohydrin reactant, preferably

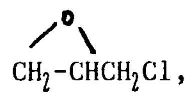

and a polar solvent and heating the mixture obtained to a temperature in the range 70 to 90°C.

Generally speaking the reaction is completed within 15 to 24 hours thus providing a process for the production of low-molecular weight alkyl azide-substituted hydroxy-terminated aliphatic polyethers such as GAP which is less time consuming and more cost effective than the Frankel process.

Preferably the epichlorohydrin reactant is

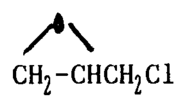

thus giving rise to product polyethers of the formula:

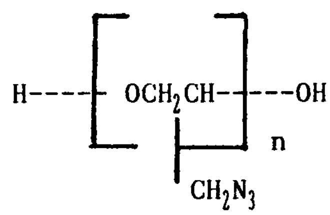

wherein is an integer from 5 to 10, and having an average molecular weight of about 500.

The preferred azide used in the reaction is sodium azide. Preferably the weight ratio of sodium azide to epichlorohydrin is about 1:1.

Suitable polar organic solvents include dimethyl formamide (DMF), and dimethyl sulfoxide (DMSO), and preferably a small amount of ethylene glycol (EG) is added to the reaction mixture as an initiator. The solvent: epichlorohydrin weight ratio is preferably in the range 0.8:1 to 2:1.

The reaction is generally carried out at a temperature in the range 70 to 90°C, most preferably about 90°C, and with agitation of the reaction mixture.

More preferably, the initial exothermic reaction is allowed to proceed at a temperature of about 70°C, followed by heating to about 90°C to complete the reaction. Specifically, the exothermic reaction arises from the opening of the epoxide ring of ECH which is caused by sodium azide and proceeds for about thirty minutes. The "30 minutes" period is approximate and depends on the duration of the gradual addition of sodium azide to the mixture ECH/DMF/EG. It is preferable to heat the reaction mixture at 70°C (approx) during the addition of $NaN_3$ in order to control the exothermic reaction. Once the sodium azide addition is over and no sudden rise in temperature is observed, then heating to 90°C starts.

At the end of the reaction, the reaction mixture is preferably cooled to obtain a crude reaction product which is then washed with water to remove DMF, EG, unreacted sodium azide and by-product sodium chloride and to recover the product polyether. Three washes with hot water (60°C) have been found appropriate.

Preferably, the washing step is followed by a purification step which involves dissolving the product polyether in a suitable polar organic solvent such as methylene chloride, drying over magnesium sulfate, and passing though a column containing silica gel. The solvent is then driven off by heating.

As shown in Table I below, the synthesis of GAP according to the invention can be accomplished at about 90°C in about 15 hours or in 24 hours by using less solvent (DMF/ECH = 0.8). At a reaction temperature lower than 90°C, the quantitative conversion of ECH to GAP will require a much longer reaction time, e.g. 48 hours at 80°C and 170 hours at 70°C.

TABLE 1

| T (°C) | Reaction Time (hours) | (DMF) (ECH) |
|---|---|---|
| 90 | 15 | 2.0 |
| 90 | 24 | 0.8 |
| 80 | 48 | 2.0 |
| 70 | 170 | 2.0 |

Example

Sodium azide (10g) is gradually added to a mixture of ECH (10g), DMF (20g) and EG (1g); heating at approximately 70°C and agitation started. Because of an initial exothermic reaction, the temperature is controlled during the first 30 minutes (approx) of the synthesis. Once the addition of sodium azide is over and no sudden rise in temperature is observed, then the reaction mixture is heated to 90°C and the agitation is carried out at this temperature for about 15 hours. Heating and agitation are stopped and the reaction mixture is allowed to cool. The polymer is given three 50 ml hot water (60°C) washes to remove DMF, EG and the salts (sodium azide and sodium chloride). The polymer is dissolved in 75 ml methylene chloride (MC). The MC solution is dried over magnesium sulfate and then is passed through a column containing 5g of silica gel. The resultant solution is heated to 50°C to remove MC and then stripped in vacuo to yield 8g (80%) of the GAP polymer: a viscous liquid with an amber colour. The GAP was characterized and had the following properties:

Elemental Analysis

C(35.7); H(5.0); N(30.9); O(18.7); C1(0.7) wt%
Nitrogen and chloride analysis of the polymer confirmed that quantitative conversion of ECH to GAP was achieved.

Infrared Spectrum

The IR absorption spectrum of the polymer showed peaks at 4.8 and 8.0 mu (characteristics of azides).

| | |
|---|---|
| Weight Average Molecular Weight (Mw) | 540 |
| Number Average Molecular Weight (Mn) | 400 |
| Equivalent Weight (Me) | 314 |
| Glass Transition Temperature (Tg) | -70°C |
| Density | 13 g/ml |
| Hydroxyl Functionality (Mn/Me) | 1.3 |

Other similar low molecular weight hydroxy-terminated aliphatic polyethers having alkyl azide substituents may be prepared in a similar manner by employing other suitable epichlorohydrins as the starting material.

**Claims**

**1.** A process for the preparation of low molecular weight alkyl azide-substituted hydroxy-terminated aliphatic polyethers of from 5 to 10 ether units in the chain which comprises reacting an alpha-epichlorohydrin with sodium, lithium or potassium azide in the presence of a polar solvent, characterised in that the process comprises adding the azide slowly to a reaction mixture containing the epichlorohydrin reactant and the polar solvent, and heating the mixture obtained to a temperature in the range 70 to 90°C for a period sufficient to complete the reaction.

**2.** A process according to claim 1, characterised in that, during the addition of the azide, the reaction mixture is heated to about 70°C to control the initial exotherm the reaction mixture thereafter being subsequently heated to about 90°C to complete the reaction.

3. A process according to claim 1 or 2, characterised in that following the addition of the azide, the reaction mixture is heated for a period of from 15 to 24 hours to complete the reaction.

4. A process according to any one of claims 1 to 3, characterised in that the polar solvent is dimethyl formamide or dimethyl sulfoxide.

5. A process according to any one of claims 1 to 4, characterised in that sufficient azide is added to the reaction mixture to provide an azide: epichlorohydrin ratio of about 1:1.

6. A process according to any one of claims 1 to 5, characterised in the weight ratio of polar solvent to epichlorohydrin in the reaction mixture is from 0.8:1 to 2:1.

7. A process according to any one of claims 1 to 6, characterised in that the reaction mixture additionally contains ethylene glycol as an initiator.

8. A process according to any one of claims 1 to 7, characterised in that at the end of the reaction the reaction mixture is cooled to obtain a crude reaction product and the product polyether is recovered therefrom by washing the crude reaction product in water.

9. A process according to claim 8, characterised in that, after washing the crude reaction product with water, the polymer is further purified by dissolving the washed polymer in a polar organic solvent, drying over magnesium sulfate and passing through a column containing silica gel.

10. A process according to any one of claims 1 to 9 wherein the reactant epichlorohydrin is

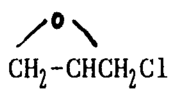

and the product is glycidyl azide polyether of the formula

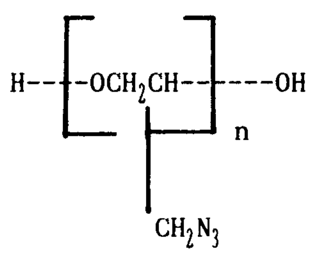

where n is an integer in the range 5 to 10.

**Revendications**

1. Procédé de préparation de polyéthers aliphatiques de faible poids moléculaire, à terminaison hydroxy et substitués par des alcoylazides, comportant 5 à 10 motifs éther dans la chaîne, qui comprend la réaction d'une alpha-épichlorhydrine avec un azide de sodium, de lithium ou de potassium en présence d'un solvant polaire, caractérisé en ce que le procédé comprend les opérations consistant à ajouter lentement l'azide à un mélange réactionnel contenant le réactif épichlorhydrine et le solvant polaire et à chauffer le mélange obtenu à une température comprise dans la plage de 70 à 90 ° C, pendant un laps de temps suffisant pour compléter la réaction.

2. Procédé selon la revendication 1, caractérisé en ce qu'on chauffe le mélange réactionnel à environ 70 ° C pendant l'addition de l'azide pour maîtriser la réaction exothermique initiale et ensuite, on chauffe le mélange réactionnel à environ 90 ° C pour compléter la réaction.

**3.** Procédé selon la revendication 1 ou 2, caractérisé en ce qu'on chauffe le mélange réactionnel pendant 15 à 24 heures après l'addition de l'azide, pour compléter la réaction.

**4.** Procédé selon l'une quelconque des revendications 1 à 3, caractérisé en ce que le solvant polaire est le diméthylformamide ou le diméthylsulfoxyde.

**5.** Procédé selon l'une quelconque des revendications 1 à 4, caractérisé en ce qu'on ajoute l'azide au mélange réactionnel en une quantité suffisante pour obtenir un rapport azide : épichlorhydrine d'environ 1:1.

**6.** Procédé selon l'une quelconque des revendications 1 à 5, caractérisé en ce que le rapport en poids solvant polaire : épichlorhydrine du mélange réactionnel est de 0,8:1 à 2:1.

**7.** Procédé selon l'une quelconque des revendications 1 à 6, caractérisé en ce que le mélange réactionnel contient en outre de l'éthylèneglycol comme initiateur.

**8.** Procédé selon l'une quelconque des revendications 1 à 7, caractérisé en ce qu'on refroidit le mélange réactionnel à l'issue de la réaction, pour obtenir un produit de réaction brut, et on en extrait le produit polyéther en lavant le produit de réaction brut dans de l'eau.

**9.** Procédé selon la revendication 8, caractérisé en ce qu'après avoir lavé le produit de réaction brut avec de l'eau, on purifie le polymère de façon complémentaire en dissolvant le polymère lavé dans un solvant organique, en le séchant sur du sulfate de magnésium et en le faisant passer à travers une colonne contenant du gel de silice.

**10.** Procédé selon l'une quelconque des revendications 1 à 10, dans lequel le réactif épichlorhydrine est

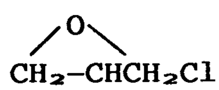

et le produit est le polyéther de glycidylazide répondant à la formule

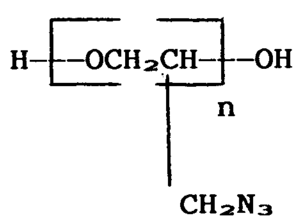

où n est un nombre entier valant de 5 à 10.

## Patentansprüche

**1.** Verfahren zur Herstellung von niedermolekularen Alkylazid-substituierten aliphatischen Polyethern mit Hydroxyendgruppen und mit 5 bis 10 Ethereinheiten in der Kette, indem man ein alpha-Epichlorhydrin mit Natrium-, Lithium- oder Kaliumazid in Gegenwart eines polaren Lösungsmittels umsetzt, **dadurch gekennzeichnet,** daß das Verfahren die langsame Zugabe des Azids zu einem den Epichlorhydrinreaktionspartner und das polare Lösungsmittel enthaltenden Reaktionsgemisch und ein Erhitzen des erhaltenen Gemisches auf eine Temperatur im Bereich von 70 bis 90 °C während ausreichender Zeit, um die Reaktion vollständig ablaufen zu lassen, umfaßt.

**2.** Verfahren nach Anspruch 1, **dadurch gekennzeichnet,** daß während der Zugabe des Azids das Reaktionsgemisch auf etwa 70 °C erhitzt wird, um die anfängliche exotherme Reaktion zu steuern, wobei das Reaktionsgemisch danach anschließend auf etwa 90 °C erhitzt wird, um die Reaktion zu Ende zu bringen.

3. Verfahren nach Anspruch 1 oder 2, **dadurch gekennzeichnet,** daß nach der Zugabe des Azids das Reaktionsgemisch während einer Dauer von 15 bis 24 h erhitzt wird, um die Reaktion zu Ende zu bringen.

4. Verfahren nach einem der Ansprüche 1 bis 3, **dadurch gekennzeichnet,** daß das polare Lösungsmittel Dimethylformamid oder Dimethylsulfoxid ist.

5. Verfahren nach einem der Ansprüche 1 bis 4, **dadurch gekennzeichnet,** daß genügend Azid zu dem Reaktionsgemisch zugegeben wird, um ein Verhältnis von Azid zu Epichlorhydrin von etwa 1:1 zu bekommen.

6. Verfahren nach einem der Ansprüche 1 bis 5, **dadurch gekennzeichnet,** daß das Gewichtsverhältnis von polarem Lösungsmittel zu Epichlorhydrin in dem Reaktionsgemisch 0,8 : 1 bis 2 : 1 beträgt.

7. Verfahren nach einem der Ansprüche 1 bis 6, **dadurch gekennzeichnet,** daß das Reaktionsgemisch zusätzlich Ethylenglycol als einen Initiator enthält.

8. Verfahren nach einem der Ansprüche 1 bis 7, **dadurch gekennzeichnet,** daß am Ende der Reaktion das Reaktionsgemisch gekühlt wird, um ein rohes Reaktionsprodukt zu erhalten, und der Produktpolyether daraus durch Waschen des rohen Reaktionsproduktion in Wasser gewonnen wird.

9. Verfahren nach Anspruch 8, **dadurch gekennzeichnet,** daß nach dem Waschen des rohen Reaktionsproduktes mit Wasser das Polymer weiter durch Auflösen des gewaschenen Polymers in einem polaren organischen Lösungsmittel, Trocknen über Magnesiumsulfat und Hindurchführen durch eine Kieselgel enthaltende Säule gereinigt wird.

10. Verfahren nach einem der Ansprüche 1 bis 9, bei dem der Reaktionspartner Epichlorhydrin

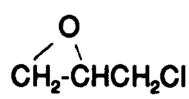

ist und das Produkt Glycidylazidpolyether der Formel

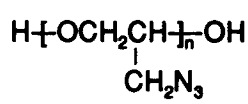

ist, worin n eine ganze Zahl im Bereich von 5 bis 10 ist.